# EUROPEAN PATENT SPECIFICATION

(11) **EP 1 729 128 B1**
(45) Date of publication and mention of the grant of the patent: **03.04.2019**
(21) Application number: 05104716.5
(22) Date of filing: 01.06.2005
(51) Int. Cl.: A61B 5/145, G01N 33/487, G06K 7/00, G01N 35/00

(54) **A coding module, a bio sensing meter and a method for operating a bio sensing meter**
Kodierungsmodul, Biosensormessvorrichtung und Verfahren zu deren Betreiben
Module de codage, mesureur à biocapteur et procédé pour sa mise en oeuvre

(43) Date of publication of application: 06.12.2006
(73) Proprietor: Bionime GmbH, 9442 Berneck (CH)
(72) Inventor: Huang, Chun-Mu, Taipei 241 (TW)
(74) Representative: Hepp Wenger Ryffel AG

(56) References cited:
- EP-A- 0 329 196
- EP-A- 0 764 469
- EP-A- 1 288 653
- WO-A1-2005/047877
- US-A- 4 714 874
- US-A- 5 053 199
- US-A- 5 281 395
- US-A- 5 366 609
- US-A- 5 873 990
- US-B1- 6 377 894

## Description

The invention relates to bio sensing meters for determining the presence of an analyte in a biological sample, and, more particularly, to a bio sensing meter whose operation is controlled by a code provided by a removably pluggable coding module. The invention further relates to a coding module pluggable into a bio sensing meter for receiving a sample strip. The coding module defines at least one code, said code ciphering at least one parameter value that is employed in controlling the operation of said meter, for example by controlling the operation of the meter.

Bio sensing meters applied for detecting substances contained in the blood to be analyzed, such as glucose or cholesterol normally employ a disposable sample strip. The sample strip has a reaction zone to allow blood placed thereon. The operation is controlled by a microprocessor. By execution of various methods, analysis results of the measurement are obtained.

It is normally necessary to calibrate instrumentation used in connection with test devices in order to compensate for variations from lot to lot on the sample strip manufactured. Various techniques have been suggested for encoding information into the sample strip, as disclosed by US 5,053,199 and references cited therein, comprising electronically encoded information on a carrier having an optical bar code, a magnetizable film, a perforated strip, a fluorogens or an electrically conductive medium on a foil.

Each of such known sample strips has to be furnished with an information code, which is an additional and thus expensive effort for a disposable device.

Another conventional sensing meter uses an additional coding module or code key designed and inserted into a receptacle similar to the slot for the sample strip. While performing a measurement, the memory key has to be inserted in the sensing meter all the time for the same batch of sample strips. According to the data or the code provided by the coding module the operation method and parameter are chosen and a correct measurement result is obtained.

US 5,366,609 and documents cited therein disclose bio sensing meters which have pluggable coding modules that enable reconfiguration of test methods and parameters employed by the meter. Threshold potentials, test times, delay periods and other pertinent test methods and constants may be entered and/or altered.

The main purpose of the coding module still is to provide information about the type of sample strip. So for each new batch of sensor strips new related information is needed.

For processing the sample measurement and the analysing routines the bio sensing meter needs certain parameter values which determine thresholds, time intervals, control numbers and calibration curve attributes.

As sample strips are disposable, preferably coding modules are disposable too. Costs for the module are therefore an important factor.

US 6,377,894 discloses an analyte test instrument having a multi purpose test port adapted to receive test strips or calibration strips. The calibration strips can include one or more memory devices such as a ROM device or a resistive calibration device with a precision resistor value.

US 5,873,990 discloses a handheld electromonitor device for rapidly measuring various analytes in environmental and biologic samples. The device comprises one slot for inserting electrode strips and calibration strips. The calibration strips may be resistor network based.

WO 2005/047877 A1 discloses an electrochemical sensing device for receiving an electrochemical sensing strip comprising a testing holder with a detachable connection to a connection slot of a main body of the sensing device. The sensing strip is able to be connected to the testing holder. Correction information for individual batches of electrochemical sensing strips may be stored on a storage component of the testing holder.

It is therefore an object of the invention to overcome the drawbacks of the prior art, especially to avoid the usage of memory IC chip technology for storing codes on coding modules, and to provide a coding module and a bio sensing meter with pluggable coding module, which have a simple design, and which can be produced cost effectively.

In accordance with the present invention a coding module is defined in claim 1, which includes at least one code, wherein the at least one code is represented by a parameter value of at least one electrical component having a determined characteristic, namely a resistor.

Electrical components can have various measurable characteristics, such as electrical characteristics, e.g. resistance, capacitance or impedance.

The advantage of using electrical characteristics of a component for encoding information is that no additional measurement device, for example an optical or magnetic detector, is necessary in the bio sensing meter. Usually the bio sensing meter provides means for performing a voltage and/or current measurement for analysing the analyte concentration, the same measurement tools can be used for reading the code. Electrical components such as resistances are not expensive.

Measuring resistances is a simple technique which is suitable for coding information, like in the present case, where e.g. only one code number or a few parameter values have to be identified.

Contrary to the state of the art IC chip technology, no integrated circuits are needed. Macroscopic electrical components can be used.

According to the invention the coding module comprises at least one resistor, which is inexpensive and can be measured easily.

Resistors are a simple implementation of non volatile information carriers.

Since a broad variety of standard resistors is available, a big number of codes can be represented only by the value of the resistances.

An even greater number of possibilities for encoding is achieved, when the at least one code is represented by a plurality of resistances, e.g. one to six, preferably four resistances.

It turned out that 2 different resistance values on four to seven places, resulting in 16 to 24 different codes, provide enough information to control the analysing method.

Preferably the code contains information regarding the sample strip batch. Samples strips should always be used with a related coding module. To reduce the error rate and to protect the slot of the bio sensing meter from pollution by the analyte or a biologic sample, the coding module has a receptacle able to accept a sample strip.

The sample strip includes a plurality of electrodes, for applying and/or measuring a voltage and/or a current.

Said coding module comprises means for establishing an electrical contact between the bio sensing meter and the sample strip.

The coding module has contacts connecting the sample strip electrodes with the bio sensing meter contacts.

According to another aspect of the invention a bio sensing meter as defined in claim 4 is provided in connection with a pluggable coding module with at least one code. The bio sensing meter has an electrical receptacle able to accept a pluggable coding module and comprises means for receiving information from said coding module defining at least one code. The code is represented by at least one electrical component having a determined resistance.

The coding module is of the above described type, wherein the code is represented by at least one, typically one to six, in particular four resistances and/or chosen from a variety of different resistances. The least one resistance can also be formed by at least one resistor.

The bio sensing meter is provided with information about the sample batch by the code on the coding module.

The code can be a simple binary code, represented by one or more resistance which is low or high, being interpreted as one of two sets of parameter values stored in the bio sensing meter or defining a binary code. Each resistance forms a bit.

The encoding can be made more complex by using a wider range of resistance values or a bigger number of resistances.

The parameter values are correlated to the value of the at least one resistance. The parameters may also be encoded by the value and the order of at least two resistances, in particular by the order of four resistance values.

In a preferred embodiment the coding module only hosts the code represented by the resistances. The value of the at least one resistance and/or the order of at least two resistances is detected by a microprocessor routine performed by said bio sensing meter.

The generation of the code based on the resistance measurements and the translation of the code into parameter values is performed by the bio sensing meter. The coding module is only a carrier of the code. The bio sensing meter has the capability of reading the code, decoding and using the information.

The code can be derived from the resistance measurement by correlating measured values, such as resistance, current or voltage, with code numbers. The code can also be formed by the resistance values.

The parameter values can be derived from the code by a microprocessor routine.

In a preferred embodiment the at least one code is decoded by extracting parameter values using a look-up table stored in a memory of the bio sensing meter.

This memory can be a read only memory. It can also be exchangeable or it can be rewritable, so that the look-up table can be exchanged or updated.

The bio sensing meter may have different receptacles for the coding module and a calibration module.

In a preferred embodiment of the invention the electrical receptacle is able to accept the coding module and is also able to accept the calibration module.

One single electrical receptacle is user friendly because less error prone, and electrical circuits can be framed more effectively.

The coding module has a receptacle able to accept sample strip. The receptacle able to accept the coding module thus is at the same time a receptacle for the sample strip, without the necessity of coming into direct contact with the sample strip.

The coding module enables electrical coupling between bio sensing meter and the sample strip.

A bio sensing system for analysing an analyte, may comprise a coding module with at least one code, preferably of the above described type and comprising a bio sensing meter, preferably of the above described type, with means for receiving the at least one code from said coding module. The code ciphers at least one parameter value that is used in controlling the operation of the bio sensing meter, for example in controlling the execution of an algorithm performed by said meter that enables determination of an analyte concentration value. The at least one code is represented by at least one electrical component having a determined resistance.

A bio sensing test set may comprise at least one test strip, and comprising a coding module with at least one code, of the above described type, pluggable into a bio sensing meter. The code ciphers at least one parameter value that is used in controlling the operation of the bio sensing meter, for example in controlling the execution of an algorithm performed by said meter that enables determination of an analyte concentration value. The at least one code is represented by at least one electrical component having a determined resistance.

Usually a bio sensing test set comprising one coding module and a plurality of samples strips form a commercial unit which is sold together in one package.

According to a further aspect of the invention there is provided a method as defined in claim 6 for operating a bio sensing meter, of the above described type, comprising the steps of
(i) inserting a coding module with at least one code into the bio sensing meter;
(ii) detecting the at least one code;
(iii) determining at least one parameter value used for controlling operation of said meter;
(iv) inserting a sample strip and adding a biologic sample;
(v) analysing the sample on the basis of the at least one parameter value;whereby the at least one code is represented by at least one electrical component having a determined characteristic, e.g. a resistance.

The invention may be more fully understood by referring to the following detailed description of illustrative embodiments of the invention and the accompanying drawings of them.
Fig. 1 is a perspective view of an example of a bio sensing meter incorporating the invention;
Fig. 2a is a perspective bottom view of a coding module according to the invention;
Fig. 2b is a perspective top view of a coding module according to the invention;
Fig. 3a is an exploded top view of a coding module according to the invention;
Fig. 3b is an exploded bottom view of a coding module according to the invention;
Fig. 4 shows the relation between analyte concentration and a measuring current;
Fig. 5a is a schematic representation of a first embodiment of the present invention;
Fig. 5b to 5c : are different tables showing coding information;
Fig. 6a is a schematic representation of a second embodiment of the present invention;
Fig. 6b and 6c show tables with coding information ;

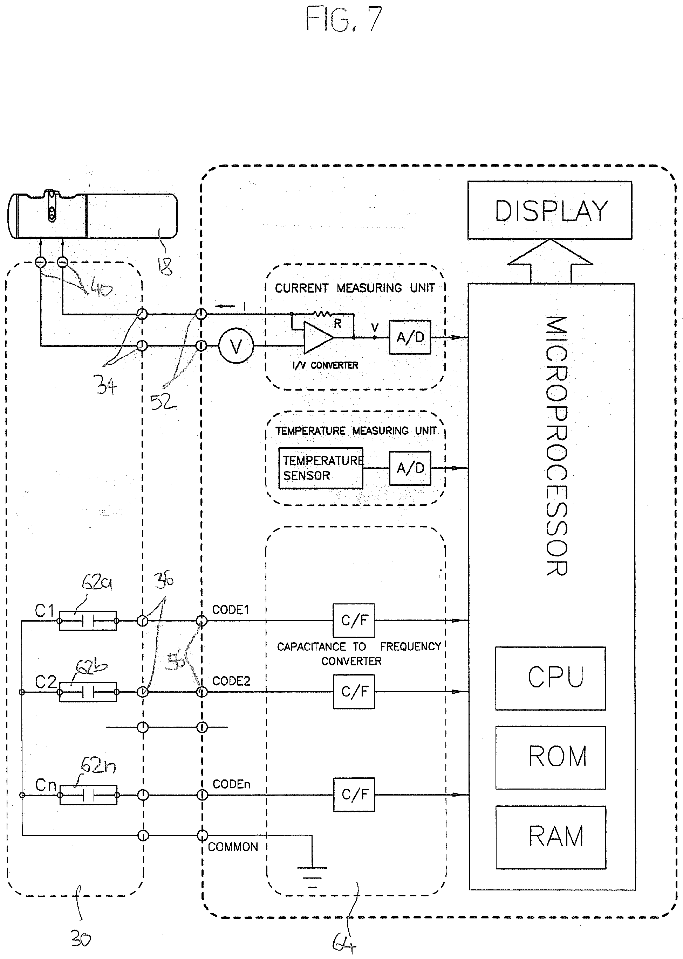

Fig. 7 shows an example not part of the present invention.

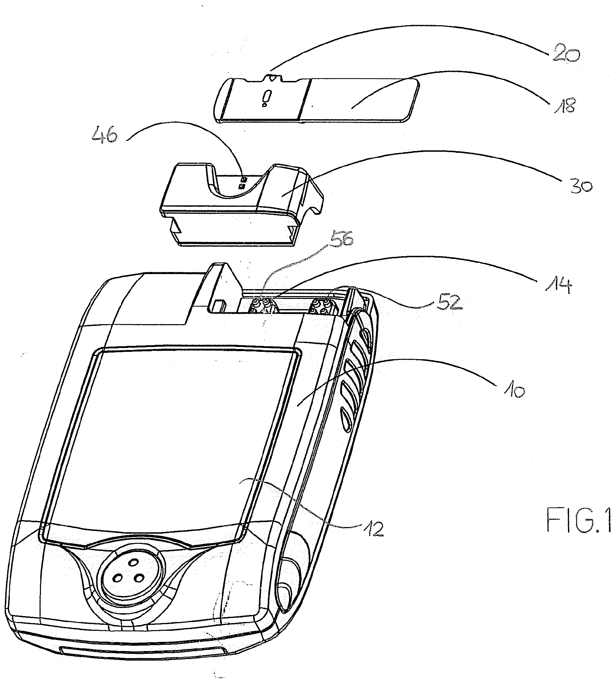

Referring to FIG. 1, a bio sensing meter 10 has a display 12, and a receptacle able to get in contact with a disposable sample strip 18. The sample strip 18 has a reaction zone which contains conductive electrodes. An enzymatic reactant layer (not shown) is formed in the reaction zone to cover the electrodes. An analyte-containing fluid, for example, a drop of blood, can be dripped on a substance entrance 20.

The bio sensing meter 10 further has a plug-in coding module 30, which is inserted into a slot 14 of the bio sensing meter 10 to be electrically connected thereto and to establish a mutual communication there between.

The coding module 30 has a receptacle 46 able to accept the sample strip 18.

The coding module 30 enables establishing an electrical connection between the bio sensing meter 10 and the sample strip 18. When the coding module 30 is plugged into the slot 14 of the bio sensing meter 10, contacts 52 of the bio sensing meter 10 get into electrical contact with the electrodes of the sample strip 18 inserted in the module 30.

The coding module 30 contains electrical components not explicitly shown in Figure 1, which are connectable to contacts 56 of the bio sensing meter 10.

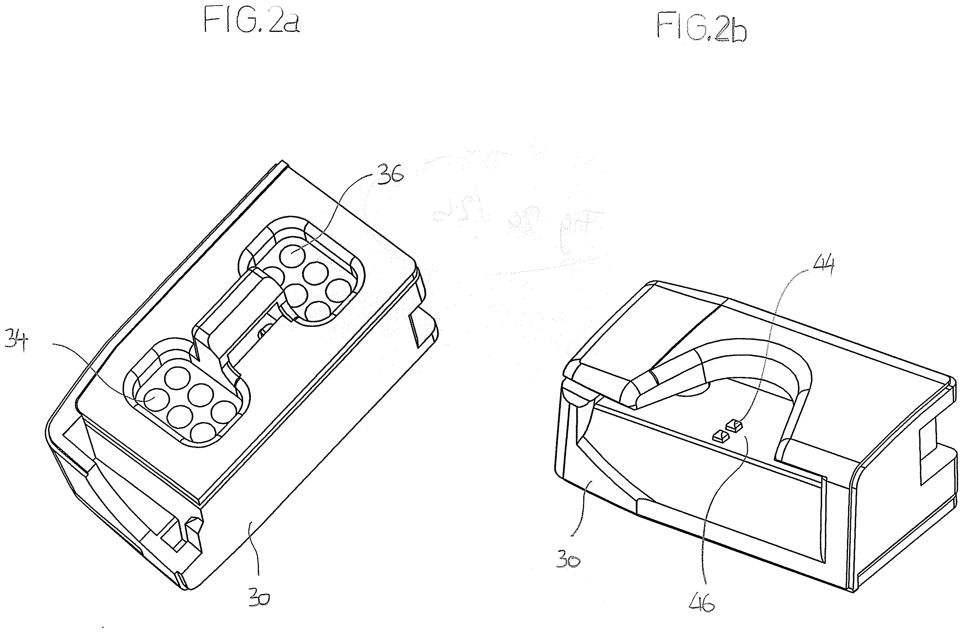

A coding module 30 is shown in Fig. 2a and 2b.

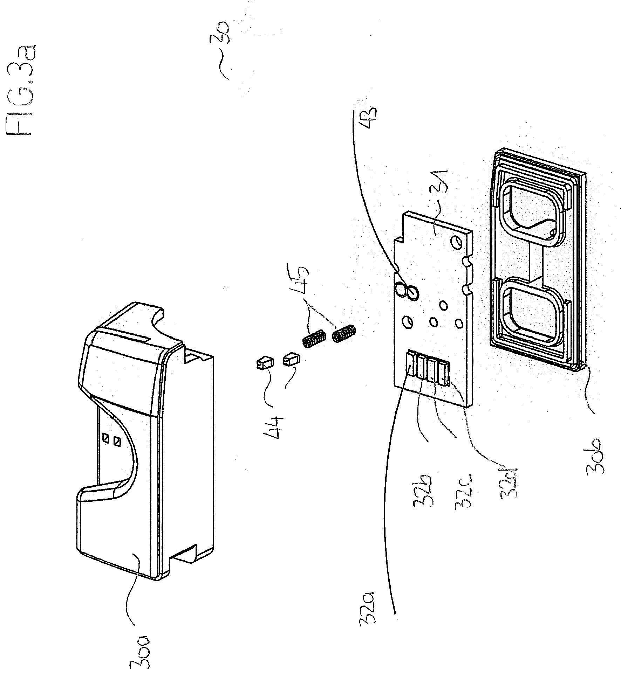

After inserting the coding module 30 into the bio sensing meter 10, the contacts 56 of the bio sensing meter 10 are in connection with contacts 36, such that the resistance values of the resistors 32, 32a, 32b, 32c, 32d (see Fig. 3a) can be detected. That is, when the bio sensing meter 10 is performing the measurement, the coding module 30 has at least to be inserted in the bio sensing meter 10 once before the measurement or permanently.

The contacts 34 of the module 30 get in contact with the contacts 52 of the meter 10 so that characteristics of the sample on the strip 30 can be measured.

The chemistries used for sample strips and analyte determination algorithms are known in the art. They will not be described in detail.

As an example, the analyte-containing sample may be a drop of blood that is subjected to a glucose determination. A disposable sample strip for a glucose determination will include, in a reaction zone, chemical reagents, basically an enzyme, for example glucose oxidase and a redox mediator, such as a potassium ferricyanide.

Figure 2b shows another perspective view of the code key 30. Within the receptacle 46 for a sample strip, there are contacts 44 in the receptacle 46 which are electrically connected to the contacts 34. Upon insertion of the sample strip 18 in the receptacle 46, the electrodes of the sample strip 18 get in contact with the contracts 44.

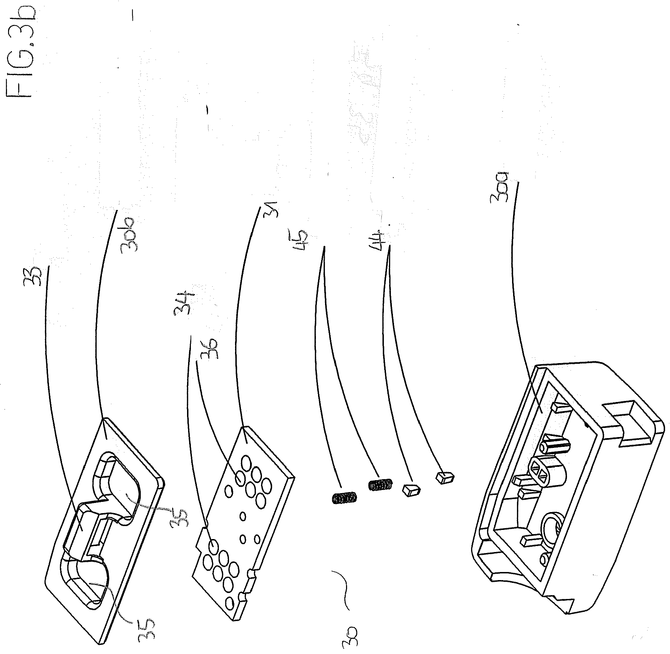

Figures 3a and 3b show an upper and lower exploded view of the coding module 30. The coding module 30 is formed by a upper part 30a and a lower part 30b. A printed circuit board 31 is arranged between these two parts. Resistors 32a, 32b, 32c, 32d are arranged on the printed circuit board 31. Resistors 32a to 32d are ciphering a code as will be described hereinafter. Contacts 44 are arranged between the printed circuit board and the upper part 30a. Springs 45 are arranged on spring contact pads 43. The springs 45 are used to hold the contacts 44 in good contact against the electrodes of the strip 18.

Figure 3b shows an exploded bottom view of the coding module 30. The bottom part 30b is provided with a lock 33 for positioning and holding the coding module 30 in the meter 10. The bottom part 30b comprises holes 35 through which the contact 34, 36 of the printed circuit board 31 may contact the contacts 52, 56 of the meter 10. The upper part 30a and the lower part 30b are formed of plastic material, typically in injection moulding.

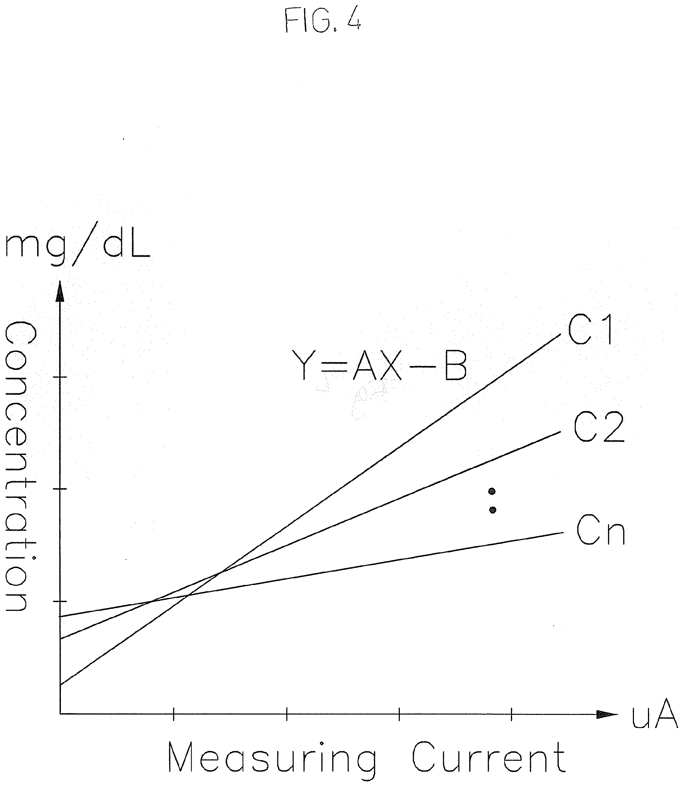

Figure 4 shows a diagram of a curve putting the concentration of the analyte in the sample, in particular glucose in the blood in relation to the measuring current determined by the meter. The concentration linearly depends on the measuring current. The concentration may be given by the formula Y=AX-B. The parameters A and B, however, depend on several conditions, in particular on the reactant composition which is used. Depending on the manufacturing process and depending on specific reactant compositions, different slopes (factor A) and different off-sets (factor B) may be applicable. The different relations are characterised by several codes C1, C2, ... Cn which are associated to specific manufacturing batches. The coding module according to the present invention in particular may be used for coding the codes C1 to Cn. It may, however, also be used for coding different analyte types or different measurement methods.

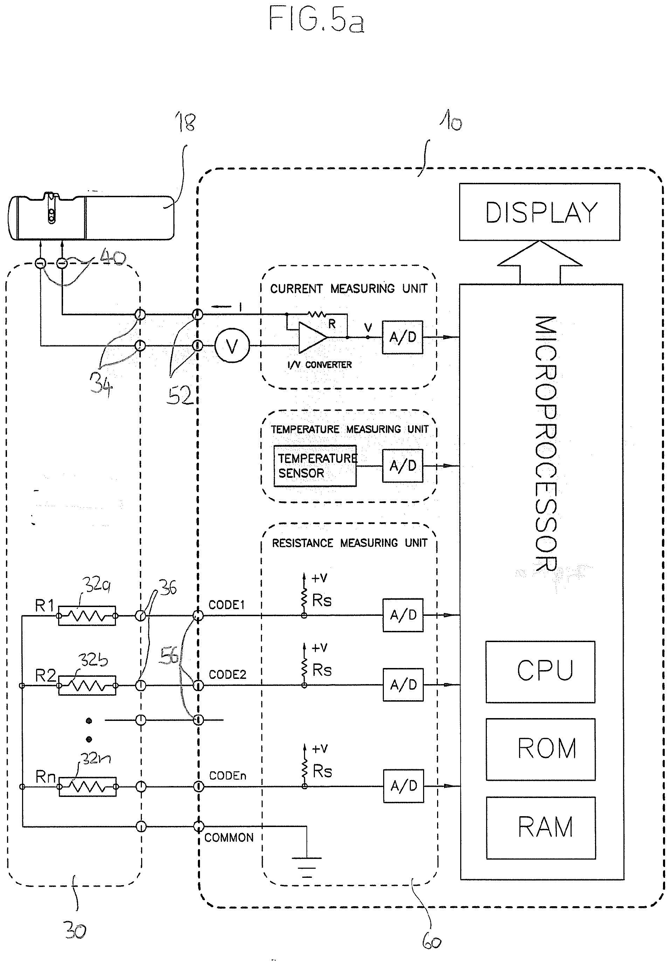

Figure 5a shows a schematic view of a meter 10 with a coding module 30 according to the present invention and with a test strip 18. The meter 10 comprises standard components such as a microprocessor with a central processing unit, a read-only memory and a random accessible memory, a display, a current measuring unit, an electrode working voltage supply unit and a temperature measuring unit. Those elements are standard in state of the art devices. In addition, the meter comprises a resistance measuring unit 60 which on the one hand is in operative connection with the microprocessor and on the other hand is connected to the contacts 56 for contacting resistors 32a, 32b, .... 32n in the coding module 30. The resistors 32a to 32n have specific resistance values R1, R2, ...Rn which cipher certain codes as will be shown hereinafter. Determination of the resistances is made in a manner known to those skilled in the art, in particular by measuring a current flowing through the resistances if a pre-defined potential is applied to the resistances. Analog/digital converters are used to transmit the resistance values to the microprocessor.

The contacts 36 on the coding module 30 get in electrical contact with the contacts 56 on the meter 10. In a similar manner, contacts 52 of the meter 10 are brought into electrical contact with the contacts 34 of the coding element 30 and consequently with the pins 40 and the electrodes of the sensor strip 18.

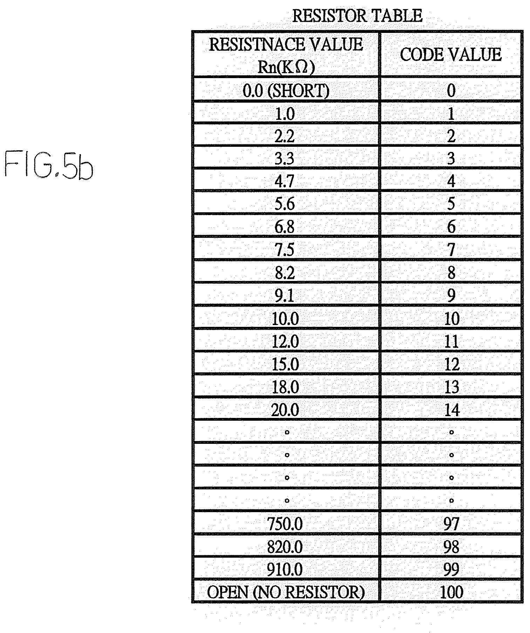

Figure 5b shows a resistor table. If only one single resistor is used, different values of this resistance may be used for defining several codes, in particular codes for different linear relationships as shown in Figure 4. Typically, one hundred different code values may be encoded with resistances in the range between 0 KΩ (short circuit) and 910 KΩ. In addition, one further code may be defined by an open circuit.

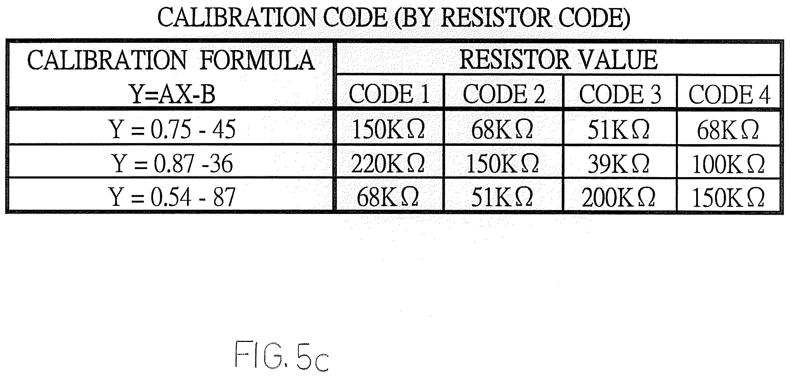

Instead of directly coding certain codes, it is also possible to code the parameter values A, B of the linear relationship as shown in Figure 4. Figure 5c shows a table where four different resistances are used for defining four codes. A quadruplet of resistance values is used to define specific values for the parameters A, B. E.g. the resistance sequence 150 KΩ/ 68 KΩ/ 51 KΩ/ 68 KΩ is used to define a slope A of 0.75 and an off set B of -45.

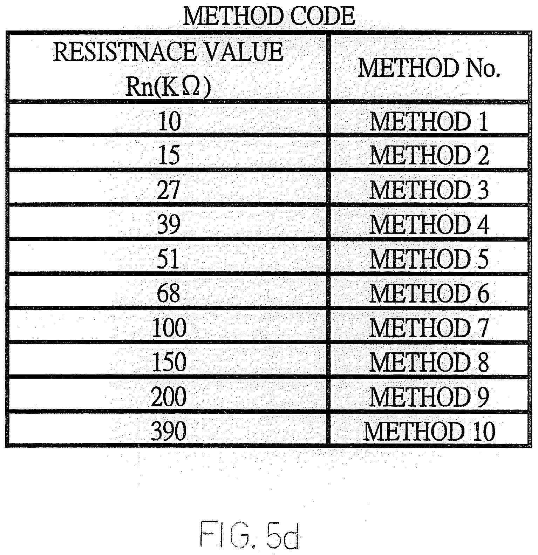

Furthermore, it is also possible to code different calculation or measurement methods. Typically, incubation times or other method parameters may vary e.g. depending on a batch of strips. For this purpose, several, e.g. ten standard measurement methods may be used. Figure 5d shows a table where different resistance values lying between 10 KΩ and 390 KΩ are used to code for one of ten several measurement methods.

The first resistor 32a shown in Figure 5a can e.g. be used for coding the values shown in Figure 5b and a second resistor 32b can be used for coding the method shown in Figure 5d.

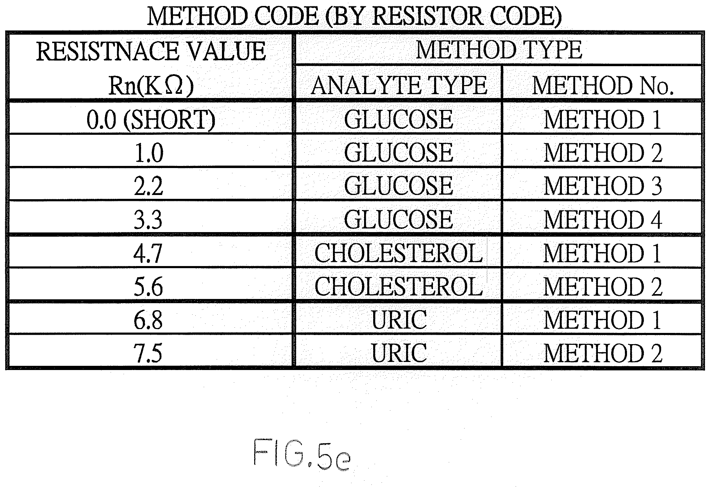

In an alternative embodiment, it is also possible to code different analyte types with a coding module 30. In the table in Figure 5e, three different analyte types are coded in context with a plurality of measurement methods by the use of eight different resistance values.

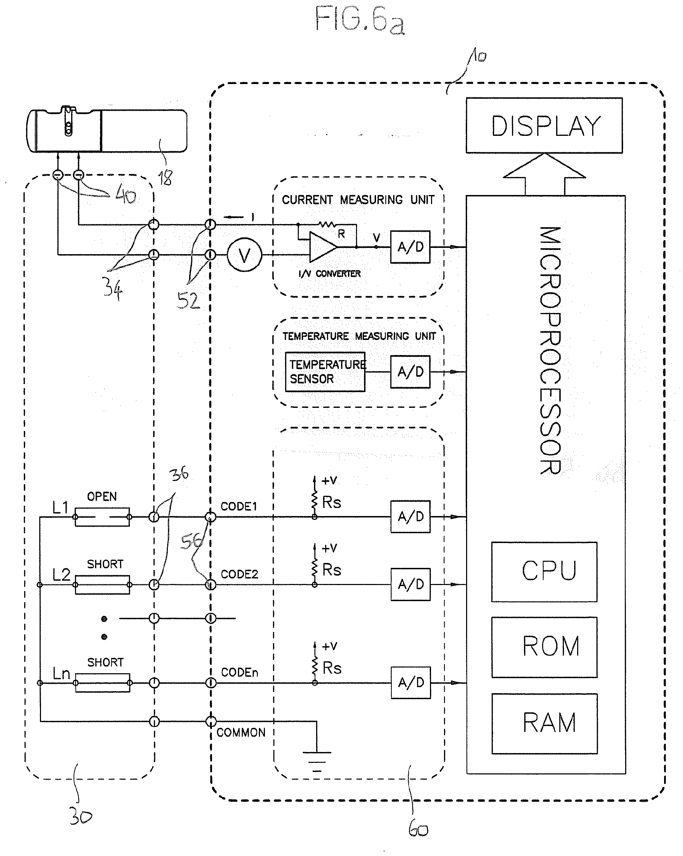

Figure 6a shows an alternative embodiment for a coding module 30. The strip 18 and the meter 10 are built identically to the one shown in Figure 5a. For coding, instead of resistances having different values, a plurality of open or short circuits are used. These open or short circuited connections L1, L2, ... Ln or switches code values such as "1" or "0" and thus form a binary code. Such open or short circuited connections my be easily arranged on the PCB layout. Determination of these resistances is made in a similar way as explained with reference to Figure 5a.

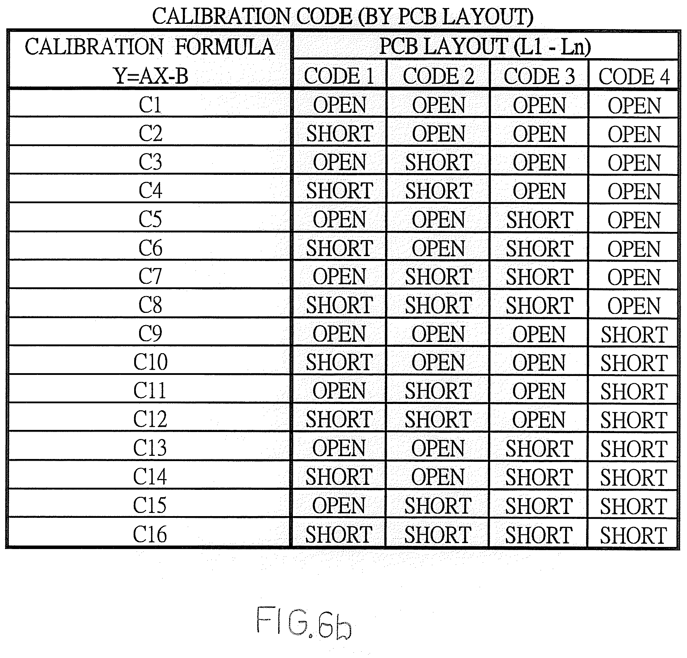

Figure 6b shows a table where different calibration formula according to 16 different codes are coded with a 4 bit arrangement of open or short circuited connections defining code 1, code 2, code 3 and code 4. Four binary codes allow for definition of 16 different calibration formula.

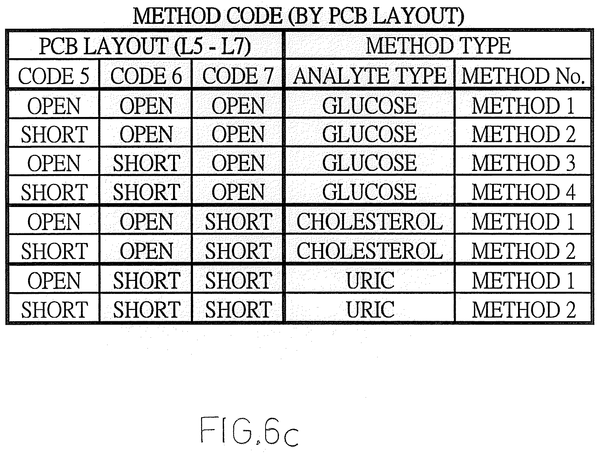

In Figure 6c, there is shown a 3 bit coding for coding different analyte types or methods. Three additional open or short circuited connections L5, L6 and L7 define six further codes which may describe eight combinations of analyte and measurement methods.

## Claims

1. A coding module connectable with
a bio sensing meter (10; 110) for receiving a sample strip (18; 118),
the coding module (30; 130) having a receptacle (46; 146) able to accept a sample strip (18; 118),
the coding module having first contacts (44) connecting electrodes of the sample strip with contacts of the bio sensing meter,
the coding module (30; 130) defining at least one code, said code ciphering at least one parameter value that is used in operation of said meter,
said code being represented by a parameter value of at least one electrical component (31) having a determined characteristic,
**characterized in that**
said at least one electrical component are resistors (32a, 32b, 32c, 32d), and **in that** the coding module (30) is formed by an upper part (30a) and a lower part (30b),
a printed circuit board (31) is arranged between the upper part (30a) and the lower part (30b), the resistors (32a, 32b, 32c, 32d) are arranged on the printed circuit board, the printed circuit board comprises second contacts (34) connected to the first contacts (44) and third contacts (36) for detecting the resistance values of the resistors (32, 32a, 32b, 32c, 32d), and the bottom part (30b) of the coding module (30) comprises holes (35) through which the second contacts (34) and the third contacts (36) of the printed circuit board (31) are contactable to the contacts of the bio sensing meter (10; 110).

2. A coding module according to claim 1, wherein the at least one resistor is chosen from a variety of different resistances in the range of 1KΩ to 1MΩ and of an open and a short circuit.

3. A coding module according to one of the claims 1 or 2, wherein the at least one code is represented by one to six, preferably four, resistances.

4. A bio sensing meter for receiving a sample strip,
said meter having an electrical receptacle (14) able to accept a coding module (30; 130) according to one of claims 1 to 3,
said bio sensing meter (10; 110) comprising means for receiving information from said coding module (30; 130) defining at least one code, which code is represented by at least one parameter value of an electrical component (31) having a resistance,
wherein said receptacle (14) comprising
contacts (52) arranged to contact the second contacts (34) of the coding module through holes (35) of the bottom part of said coding module for making an electrical connection between a sample strip (18; 118) and said meter and contacts (56) arranged to contact the third contacts (36) of the coding module through holes (35) of the bottom part of said coding module for detecting the resistance values of the resistors,
the biometer comprising a resistance measuring unit (60) and a microprocessor in operative connection with said resistance measuring unit (60) for determination of resistance values ciphering a code.

5. A bio sensing meter according to claim 4, wherein the meter (10; 110) comprises a memory having a look-up table for decoding the at least one code and/or extracting parameter values.

6. A method for operating a bio sensing meter (10; 110), according to one of claims 4 to 5, comprising the steps of
(i) inserting a coding module (30; 130), according to one of claims 1-3, with at least one code into bio sensing meter (10; 110);
(ii) detecting the at least one code;
(iii) determining at least one parameter value on the basis of said code;
(iv) analysing the sample on the basis of the at least one parameter value;
wherein the at least one code is represented by at least one electrical component (31) having a determined characteristic, namely a resistance.

## Patentansprüche

1. Kodiermodul, das mit einem Biomessgerät (10; 110) zur Aufnahme eines Teststreifens (18; 118) verbunden werden kann.
wobei das Kodiermodul (30; 130) eine Anschlussbuchse (46; 146), die in der Lage ist, einen Teststreifen (18; 118) aufzunehmen, aufweist,
wobei das Kodiermodul erste Kontakte (44), die Elektroden des Teststreifens mit Kontakten des Biomessgeräts verbinden, aufweist,
wobei das Kodiermodul (30; 130) mindestens einen Code definiert,
wobei der Code mindestens einen Parameterwert verschlüsselt, der bei dem Betrieb des Messgeräts verwendet wird,
wobei der Code durch einen Parameterwert mindestens einer elektrischen Komponente (31), die ein bestimmtes Merkmal aufweist, dargestellt wird,
**dadurch gekennzeichnet, dass**
es sich bei der mindestens einen elektrischen Komponente um Widerstände (32a, 32b, 32c, 32d) handelt und dass das Kodiermodul (30) aus einem oberen Teil (30a) und einem unteren Teil (30b) gebildet wird,
wobei eine Leiterplatte (31) zwischen dem oberen Teil (30a) und dem unteren Teil (30b) angeordnet ist, wobei die Widerstände (32a, 32b, 32c, 32d) auf der Leiterplatte angeordnet sind,
wobei die Leiterplatte zweite Kontakte (34), die mit den ersten Kontakten (44) verbunden sind, und dritte Kontakte (36) zum Detektieren der Widerstandswerte der Widerstände (32, 32a, 32b, 32c, 32d) umfasst, und der untere Teil (30b) des Kodiermoduls (30) Löcher (35), durch die die zweiten Kontakte (34) und die dritten Kontakte (36) der Leiterplatte (31) mit den Kontakten des Biomessgeräts (10; 110) kontaktierbar sind, umfasst.

2. Kodiermodul nach Anspruch 1, wobei der mindestens eine Widerstand aus einer Vielzahl verschiedener Widerstände im Bereich von 1 KΩ bis 1 MΩ und aus einem offenen Stromkreis und einem Kurzschluss ausgewählt ist.

3. Kodiermodul nach einem der Ansprüche 1 oder 2, wobei der mindestens eine Code durch einen bis sechs, bevorzugt vier, Widerstände dargestellt wird.

4. Biomessgerät zur Aufnahme eines Teststreifens,
wobei das Messgerät eine elektrische Anschlussbuchse (14), die in der Lage ist, ein Kodiermodul (30; 130) nach einem der Ansprüche 1 bis 3 aufzunehmen, aufweist,
wobei das Biomessgerät (10; 110) Mittel zum Empfangen von Informationen von dem Kodiermodul (30; 130), die mindestens einen Code definieren, umfasst, wobei der Code durch mindestens einen Parameterwert einer elektrischen Komponente (31), die einen Widerstand aufweist, dargestellt wird,
wobei die Anschlussbuchse (14) Folgendes umfasst:
Kontakte (52), die angeordnet sind, die zweiten Kontakte (34) des Kodiermoduls durch Löcher (35) im unteren Teil des Kodiermoduls zu kontaktieren, um eine elektrische Verbindung zwischen einem Teststreifen (18; 118) und dem Messgerät herzustellen, und
Kontakte (56), die angeordnet sind, die dritten Kontakte (36) des Kodiermoduls durch Löcher (35) im unteren Teil des Kodiermoduls zu kontaktieren, um die Widerstandswerte der Widerstände zu detektieren,
wobei das Messgerät eine Widerstandsmesseinheit (60) und einen Mikroprozessor in operativer Verbindung mit der Widerstandsmesseinheit (60) zur Bestimmung der Widerstandswerte, die einen Code verschlüsseln, umfasst.

5. Biomessgerät nach Anspruch 4, wobei das Messgerät (10; 110) einen Speicher, der eine Nachschlagetabelle zur Decodierung des mindestens einen Codes und/oder zum Extrahieren von Parameterwerten aufweist, umfasst.

6. Verfahren zum Betreiben eines Biomessgeräts (10; 110) nach einem der Ansprüche 4 oder 5, umfassend die folgenden Schritte:
(i) Einstecken eines Kodiermoduls (30; 130), nach einem der Ansprüche 1 bis 3, mit mindestens einem Code in das Biomessgerät (10; 110);
(ii) Detektieren des mindestens einen Codes;
(iii)Bestimmen mindestens eines Parameterwertes auf Grundlage des Codes;
(iv) Analysieren der Probe auf der Grundlage des mindestens einen Parameterwertes;
wobei der mindestens eine Code durch mindestens eine elektrische Komponente (31), die ein bestimmtes Merkmal, nämlich einen Widerstand, aufweist, dargestellt wird.

## Revendications

1. Module de codage connectable avec
un mesureur à biocapteur (10 ; 110) pour recevoir une bande d'échantillon (18 ; 118),
le module de codage (30 ; 130) ayant un réceptacle (46 ; 146) capable d'accepter une bande d'échantillon (18 ; 118),
le module de codage ayant des premiers contacts (44) connectant des électrodes de connexion de la bande d'échantillon aux contacts du mesureur à biocapteur,
le module de codage (30 ; 130) définissant au moins un code,
ledit code chiffrant au moins une valeur de paramètre qui est utilisée dans le fonctionnement dudit mesureur,
ledit code étant représenté par une valeur de paramètre d'au moins un composant électrique (31) ayant une caractéristique déterminée,
**caractérisé en ce que**
ledit au moins un composant électrique sont des résistances (32a, 32b, 32c, 32d), et **en ce que** le module de codage (30) est formé par une partie supérieure (30a) et une partie inférieure (30b),
une carte de circuit imprimé (31) est disposée entre la partie supérieure (30a) et la partie inférieure (30b), les résistances (32a, 32b, 32c, 32d) sont disposées sur la carte de circuit imprimé,
la carte de circuit imprimé comprend des deuxièmes contacts (34) connectés aux premiers contacts (44) et des troisièmes contacts (36) pour détecter les valeurs de résistance des résistances (32, 32a, 32b, 32c, 32d), et la partie inférieure (30b) du module de codage (30) comprend des trous (35) par lesquels les deuxièmes contacts (34) et les troisièmes contacts (36) de la carte de circuit imprimé (31) peuvent entrer en contact avec les contacts du mesureur à biocapteur (10, 110).

2. Module de codage selon la revendication 1, l'au moins une résistance étant choisie parmi différentes résistances dans la plage de 1 KΩ à 1 MΩ et parmi un circuit ouvert et un court-circuit.

3. Module de codage selon l'une des revendications 1 ou 2, l'au moins un code étant représenté par une à six, de préférence quatre, résistances.

4. Mesureur à biocapteur pour la réception d'une bande d'échantillon,
ledit mesureur ayant une réceptacle électrique (14) pouvant recevoir un module de codage (30 ; 130) selon l'une des revendications 1 à 3,
ledit mesureur à biocapteur (10 ; 110) comprenant des moyens pour recevoir des informations dudit module de codage (30 ; 130) définissant au moins un code, lequel code est représenté par au moins une valeur de paramètre d'un composant électrique (31) ayant une résistance,
ledit réceptacle (14) comprenant :
des contacts (52) agencés pour mettre en contact les deuxièmes contacts (34) du module de codage à travers des trous (35) de la partie inférieure dudit module de codage pour établir une connexion électrique entre une bande d'échantillon (18 ; 118) et ledit mesureur et
des contacts (56) agencés pour mettre en contact les troisièmes contacts (36) du module de codage à travers des trous (35) de la partie inférieure dudit module de codage pour détecter les valeurs de résistance des résistances,
ledit mesureur comprenant une unité de mesure de résistance (60) et un microprocesseur en liaison opérationnelle avec ladite unité de mesure de résistance (60) pour déterminer des valeurs de résistance chiffrant un code.

5. Mesureur à biocapteur selon la revendication 4, le mesureur (10 ; 110) comprenant une mémoire ayant une table de recherche pour décoder l'au moins un code et/ou extraire des valeurs de paramètres.

6. Procédé de fonctionnement d'un mesureur à biocapteur (10 ; 110), selon l'une des revendications 4 à 5, comprenant :
(i) l'insertion d'un module de codage (30 ; 130), selon l'une des revendications 1 à 3, avec au moins un code dans un mesureur à biocapteur (10 ; 110) ;
(ii) la détection de l'au moins un code ;
(iii) la détermination d'au moins une valeur de paramètre sur la base dudit code ;
(iv) l'analyse de l'échantillon sur la base de l'au moins une valeur de paramètre ;
l'au moins un code étant représenté par au moins un composant électrique (31) ayant une caractéristique déterminée, à savoir une résistance.
